# EUROPEAN PATENT SPECIFICATION

(11) **EP 4 346 924 B1**
(45) Date of publication and mention of the grant of the patent: **02.07.2025**
(21) Application number: 22731293.1
(22) Date of filing: 01.06.2022
(51) Int. Cl.: A61L 24/00, A61L 24/02, A61L 24/04

(54) **SOL-GEL COMPOSITIONS FOR VETERINARY USE**
SOL-GEL-ZUSAMMENSETZUNGEN ZUR VETERINÄRMEDIZINISCHEN VERWENDUNG
COMPOSITIONS SOL-GEL À USAGE VÉTÉRINAIRE

(30) Priority: 03.06.2021 GB 202107976
(43) Date of publication of application: 10.04.2024
(73) Proprietor: Norbrook Laboratories Limited, Newry, Co. Down BT35 6QQ (GB)
(72) Inventor: BRANNIGAN, Tim, Newry Co. Down BT35 6QQ (GB); CLARKE, Anne, Newry Co. Down BT35 6QQ (GB); REYNOLDS, Louise, Newry Co. Down BT35 6QQ (GB)
(74) Representative: HGF
(86) International application number: PCT/GB2022/051408
(87) International publication number: WO 2022/254224

(56) References cited:
- WO-A1-2021/020535
- CN-A- 110 193 084
- US-A1- 2018 303 866
- BANDARI SURESH ET AL: "Continuous twin screw granulation - An advanced alternative granulation technology for use in the pharmaceutical industry", INTERNATIONAL JOURNAL OF PHARMACEUTICS, ELSEVIER, NL, vol. 580, 16 March 2020 (2020-03-16), XP086127094, ISSN: 0378-5173, [retrieved on 20200316], DOI: 10.1016/J.IJPHARM.2020.119215

## Description

### FIELD OF THE INVENTION

The invention relates to compositions that can undergo a sol-gel transition at a physiologically relevant temperature, and to methods for their manufacture. The invention further relates to the composition for use in therapy and/or for use as medicament, preferably in the treatment of mastitis.

### BACKGROUND

Mastitis is an inflammation of the mammary gland that often involves a bacterial infection or sometimes a viral infection. In a veterinary context, mastitis is particularly problematic for cattle, especially dairy cows, because it can become contagious within the confines of a farm. It can lead to large economic losses of production for farmers, particularly in the dairy industry. Due to the harmful effects of the pathogens and/or the subsequent veterinary treatment used, the milk may be unfit for human consumption. Unchecked cases can also cause permanent damage to the udders of the animals with severe cases potentially ending in death.

To prevent mastitis, teat seals have been developed to form a seal that provides a physical barrier to prevent entry of microbes into the teat or udder. The teat seal may be an external teat seal, which provides an external physical barrier across the entrance to the teat canal.

Typically, external teat seals are acrylic, latex or polymer-based and are applied after milking has finished and at the beginning of the dry period. When external teats seals are applied only once during the dry period, they do not provide sufficient protection for the whole of this period. It may be necessary for external teat seals to be applied multiple times during the dry period or, at the very least, the status of the seal needs to be checked frequently to ensure it has not failed.

Internal teat seals are typically thick pastes that are infused into the udder to block or seal the teat canal or to plug the teat cistern. The teat seal forms a physical barrier, either in the form of a paste or when it solidifies, within the teat canal, thereby preventing the entry of problematic microbes. Internal teat seals commonly include a heavy metal, such as bismuth in the form of bismuth subnitrate (Bi₅O(OH)₉(NO₃)₄, mixed into a gel base. The gel base may include polyethylene glycol and liquid paraffin as a vehicle.

In general, currently available teat seals are not sufficiently long-lasting and can be difficult to apply and/or remove. An example of an internal teat sealant is disclosed in US 2018/303866 A1. Many teat seals also contaminate milk or dairy products Heavy metal containing teat seals, particularly bismuth-containing teat seals, contaminate dairy products and are known to be the cause of black spot defects (BSDs) in aged cheese. Such BSDs can result in a dramatic reduction in the value of the cheeses that are affected. For example, The Dairy Grading Branch of the U.S. Department of Agriculture (USDA) Agricultural Marketing Services has deemed cheese with BSD as "No U.S. Grade Assigned Due to Foreign Material", which renders the product ineligible for USDA grade certification.

### SUMMARY OF THE INVENTION

The invention provides a composition comprising (a) 15.0 % w/w to 25.0 % w/w of a thermoresponsive polymer and (b) 0.75 % w/w to 2.00 % w/w of a viscosity modifier. The thermoresponsive polymer is a polyvinyl caprolactam-polyvinyl acetate-polyethylene glycol graft copolymer and the viscosity modifer is a cross-linked poly(acrylic acid).

The composition of the invention is particularly suitable for use as a teal seal, particularly an internal teat seal. Thus, the composition may be a teat seal.

The invention also provides a method of manufacturing the composition. The method comprises mixing a suspension of a thermoresponsive polymer with a viscosity modifier.

The invention further provides a system for administering the composition to a mammary gland of an animal. The system comprises (i) an application device and/or an infusion device, and (ii) the composition as defined herein.

A further aspect of the invention relates to a kit of parts comprising (a) the composition as defined herein together with instructions for simultaneous, concurrent, separate or sequential use in combination with (b) a therapeutic agent, such as defined herein.

The invention further relates to a package comprising (a) the composition as defined herein and (b) a therapeutic agent, such as defined herein, for simultaneous, concurrent, separate or sequential use in the treatment or prevention of mastitis.

The invention also relates to the composition as defined herein for use in therapy and/or for use as a medicament.

The invention further provides to the composition as defined herein for use in the treatment or prevention of mastitis.

Also provided by the invention is the use of the composition as defined herein for the manufacture of a medicament for the treatment or prevention of mastitis.

Another aspect of the invention is the composition as defined herein for use in a method of treating or preventing mastitis wherein said method comprises administering a composition as defined herein to a mammary gland of an animal.

Another aspect of the invention relates to a composition as defined herein for use in the treatment or prevention of mastitis in combination with simultaneous, concurrent, separate or sequential administration of (b) a therapeutic agent, such as defined herein.

The invention further relates to the use of the composition as defined herein for the manufacture of a medicament for simultaneous, concurrent, separate or sequential use in combination with (b) a therapeutic agent, such as defined herein, for the treatment or prevention of mastitis.

A further aspect of the invention relates to a therapeutic agent, such as defined herein, for use in the treatment or prevention of mastitis in combination with simultaneous, concurrent, separate or sequential administration of (a) a composition as defined herein.

The invention further relates to the use of a therapeutic agent, such as defined herein, for the manufacture of a medicament for simultaneous, concurrent, separate or sequential use in combination with (a) a composition as defined herein, for the treatment or prevention of mastitis.

### BRIEF DESCRIPTION OF FIGURES

The invention is further described hereinafter with reference to the accompanying drawing.

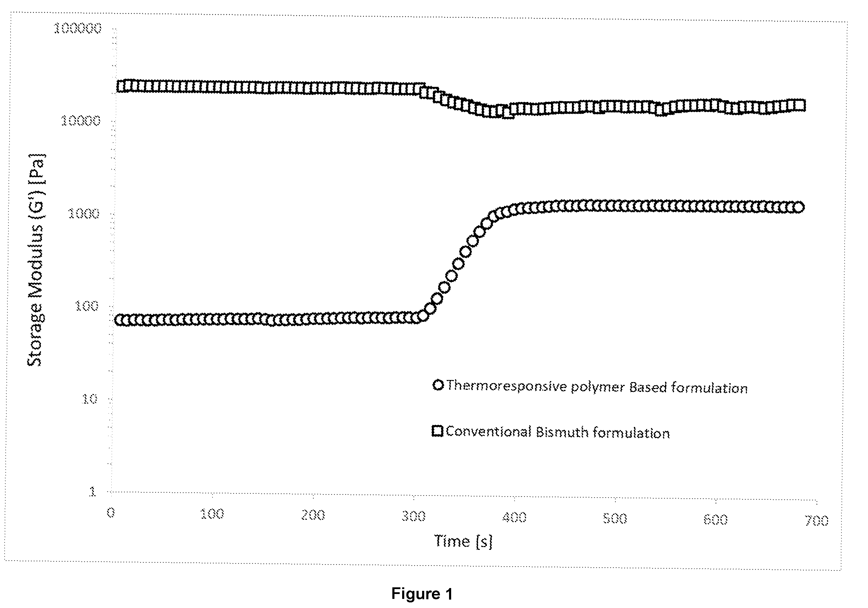

Figure 1 is a graph showing the variation in storage modulus (G') over time for a composition in accordance with the invention and for a conventional bismuth-containing formulation.

### DETAILED DESCRIPTION

The term "about" as used herein in conjunction with a measurable value, such as an amount or a period of time, encompasses reasonable variations of the value, for instance, to allow for experimental error in the measurement of said value.

As used herein, the term "comprises" has an open meaning, which allows other, unspecified features to be present. This term embraces, but is not limited to, the semi-closed term "consisting essentially of" and the closed term "consisting of". Unless the context indicates otherwise, the term "comprises" may be replaced with either "consisting essentially of" or "consists of". The term "consisting essentially of" may be replaced with "consists of".

In principle, the composition of the invention can be used in a variety of medicinal applications, including both human and veterinary applications, because of its advantageous properties.

The composition of the invention is particularly suitable for administration to a mammary gland of an animal.

The term "mammary gland" as used herein refers to any part of the breast or udder of an animal, including the secreting tissue, connective tissue, gland(s) or teat(s). In the context of administering or applying the composition or a therapeutic agent to a mammary gland, it is preferred that the mammary gland refers to the teat, particularly the teat canal or teat cistern.

The term "animal" as used herein generally refers to an animal having a mammary gland, such as a female animal. It is preferred that the term "animal" refers to a mammal, including humans. More preferably, the mammal is a female mammal, particularly a female non-human mammal.

The term "teat seal" as used herein refers to a composition used to form a physical barrier on the surface of or inside an animal teat. A teat sealant can be on the teat surface, inside the teat streak canal, and/or inside the teat cistern.

The composition of the invention is particularly suitable for administration to a mammary gland of an animal, preferably a female animal. The animal may be a non-human animal. The composition is preferably for administration to a mammary gland of a female, non-human mammal.

In general, the composition is a liquid suspension at ambient temperatures (i.e. the composition is in the form of a liquid suspension), preferably the composition is a sol at ambient temperatures (i.e. the composition is in the form of a sol). The term "ambient temperatures" as used herein refers to a temperature range of from about 5°C to about 30°C, preferably from about 10°C to about 30°C. In some locations, the normal "ambient temperature" may fall outside these ranges.

The composition of the invention can be dispensed or administered by a syringe. At ambient temperatures, such as room temperature, the composition has a fluid-like consistency and can easily be administered using, for example, a single-barrel syringe. Its rheological properties at ambient temperatures render it particularly suitable for use as a teat seal. The composition of the invention may be easier to syringe than many commercially available products, particularly internal teat seal formulations.

The composition may have a tan δ (at 25°C), where tan δ ≥ 0.50. It is preferred that the composition has a tan δ (at 25°C), where tan δ ≥ 0.75, more preferably tan δ ≥ 0.95, even more preferably tan δ ≥ 1.00.

The parameter "tan δ" as used herein refers to the tangent of the phase angle, i.e. the ratio of the viscous to elastic response of a viscoelastic material. This parameter is defined as the ratio between the elastic modulus (G') and the loss modulus (G") (e.g. tan δ = G"/G'). It provides a quantifier of the presence and the degree of elasticity in a fluid. The tan δ (at 25°C) provides a measure of the fluidity of the composition at an ambient temperature. At 25°C liquids have a tan δ > 1.

The values of elastic and loss moduli (and, hence, tan δ) as a function of time at a constant stress of 1.0 Pa (a value in the plateau region of LVE) and frequency (f = 0.1 Hz) were measured using parallel plate rheometer fitted with a 40 mm geometry at a gap of 1000 µm. Testing was performed with a solvent trap loaded with water in place to prevent sample drying. In the first step of the experiment (5 mins), the temperature was kept at 25°C. In the second step, the temperature was increased at approximately 10°C/min to 37°C. In the final step (5 - 10 min), the temperature was fixed at 37°C. Values of the elastic and loss moduli were calculated from the obtained at 20 second intervals across the experimental range.

Typically, the composition undergoes a physicochemical change at a physiological temperature, such as around a typical body temperature (e.g. at about 37.5°C).

The term "physiological temperature" as used herein refers to a temperature greater than or equal to about 32°C, such as greater than or equal to about 35°C. It is preferred that the temperature is from about 32°C to about 40°C, preferably from about 34°C to about 37°C. In some physiologies or certain physiological locations, such as away from the body (e.g. an extremity), the normal physiological temperature may fall outside these ranges.

Generally, the physicochemical change at the physiological temperature is a sol-gel transition (e.g. a phase transition between a sol phase and a gel phase). A sol-gel transition refers to the change from a liquid state to a gel state. The occurrence of a sol-gel transition can be determined by rheological measurements. For example, as understood in the art, the elastic modulus (also known as "Young's modulus" or the storage modulus (G')) increases substantially when there is a change from the liquid state to the gel state.

Thus, the composition of the invention may undergo a sol-gel transition at a physiological temperature. The sol-gel transition may be thermo-reversible or thermo-irreversible, such as at the physiological temperature. It is preferred that the sol-gel transition is thermo-irreversible, such as at the physiological temperature. These properties result from the inclusion of the thermoresponsive polymer.

The composition of the invention may have a storage modulus (G') at 37°C of at least 450 Pa. It is preferred that the composition has a storage modulus (G') at 37°C of at least 700 Pa, more preferably at least 950 Pa, such as at least 1200 Pa, and even more preferably at least 1400 Pa.

The composition is typically a gel at the physiological temperature, preferably the composition is a solid gel or a semi-solid gel at the physiological temperature. The term "solid" refers to a physical form (e.g. a gel) that is non-flowable at the physiological temperature. The term "semi-solid" refers to a physical form (e.g. a gel) that is not solid, but is sufficiently viscous to resist flowing at the physiological temperature.

The composition may have a tan δ (at 37°C) of less than or equal to 0.70. It is preferred that the composition has a tan δ (at 37°C) of less than or equal to 0.55, more preferably less than or equal to 0.50, such as less than or equal to 0.45, and even more preferably less than or equal to 0.40.

An advantageous property of the composition is that at ambient temperatures, such as room temperature, it has a fluid-like consistency and can easily be administered using, for example, a single-barrel syringe. After administration to an animal, the composition can undergo a sol-gel transition at a physiological temperature, such as in the mammary gland of an animal, to transform the composition into a viscoelastic solid. The resulting solidified composition can form a physical barrier within the mammary gland and can act as a teat seal. This physical barrier prevents the entry into the mammary gland of mastitis-causing pathogens. It can also seal the mammary gland to avoid exacerbation of any pre-existing infection, thereby allowing separate treatment of the infection and/or the animal's immune system to clear it up.

The composition of the invention is a stable product, which can solidify rapidly at a physiological temperature. It can remain solid after administration, forming a strong and durable seal that remains effective over a set period of time. The seal does not leak or drip after application. In many instances, it may be necessary for the composition to be applied in a single application to provide a seal that will last for the entirety of the required duration.

In principle, a thermoresponsive polymer may be a natural polymer or a synthetic polymer. A natural polymer is a polymer that occurs in nature. A synthetic polymer is a nonnatural, human-made polymer.

When a thermoresponsive polymer is a natural polymer, then, by way of illustration, a thermoresponsive polymer may comprise, or consist essentially of, a polysaccharide, such as a chitosan, a dextran or a cellulose, or a protein, such as a gelatin or a collagen.

When a thermoresponsive polymer is a synthetic polymer, then, by way of illustration, the thermoresponsive polymer may comprise, or consist essentially of, a poly(N-isopropylacrylamide), a poly[2-(dimethylamino)ethyl methacrylate] (pDMAEMA), a poly(vinylcaprolactam), a poly-2-isopropyl-2-oxazoline, a polyvinyl methyl ether, or a poloxamer.

The composition comprises a total amount of about 15.0 % w/w to about 25.0 % w/w, more preferably a total amount of about 17.5 % w/w to about 22.5 % w/w of the thermoresponsive polymer.

The thermoresponsive polymer is a polyvinyl caprolactam-polyvinyl acetate-polyethylene glycol graft copolymer, particularly a poly(N-vinyl caprolactam)-poly(vinyl acetate)-poly(ethylene glycol) graft copolymer.

In general, the composition comprises the thermoresponsive polymer, where the thermoresponsive polymer is polydisperse. Thus, the composition comprises a distribution of thermoresponsive polymer molecules by molecular weight.

Typically, the thermoresponsive polyvinyl caprolactam-polyvinyl acetate-polyethylene glycol graft copolymer (e.g. a poly(N-vinyl caprolactam)-poly(vinyl acetate)-poly(ethylene glycol) graft copolymer), has a weight average molecular weight of from 50000 to 350000 g.mol⁻¹, preferably from 75000 to 250000 g.mol⁻¹, such as from 75000 to 175000 g.mol⁻¹, more preferably from 80000 to 150000 g.mol⁻¹, particularly 90000 to 140000 g.mol⁻¹. The weight averaged molecular weight can be measured by gel permeation chromatography (e.g. calibrated using a polystyrene standard).

Generally, the thermoresponsive polymer, particularly the polyvinyl caprolactam-polyvinyl acetate-polyethylene glycol graft copolymer (e.g. a poly(N-vinyl caprolactam)-poly(vinyl acetate)-poly(ethylene glycol) graft copolymer), has a hydrophilic-lipophilic balance (HLB) of about 12 to about 16, preferably about 13 to about 15 (e.g. about 14). The HLB is determined using the Davies method known in the art.

The poly(N-vinyl caprolactam)-poly(vinyl acetate)-poly(ethylene glycol) graft copolymer may be represented by formula (1), 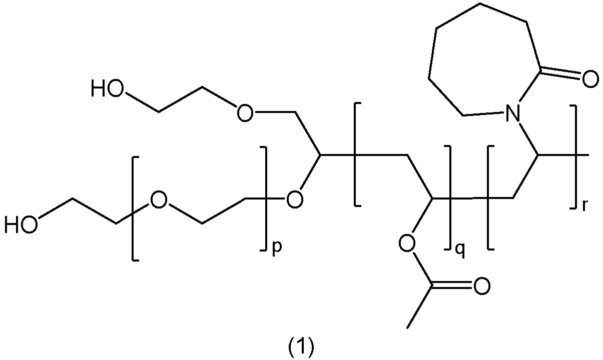 where each of p, q and r is an integer greater than 0.

As can be seen from formula (1) above, the poly(N-vinyl caprolactam)-poly(vinyl acetate)-poly(ethylene glycol) graft copolymer comprises, or consists essentially of, a first polymer unit, a second polymer unit and a third polymer unit. The first polymer unit comprises a polyethylene glycol, preferably a polyethylene glycol 6000 (PEG 6000). The second polymer unit comprises a poly(vinylcaprolactam), particularly a poly(N-vinyl caprolactam). The third polymer unit comprises a polyvinyl-acetate.

Typically, the poly(N-vinyl caprolactam)-poly(vinyl acetate)-poly(ethylene glycol) graft copolymer may obtained or is obtainable from about 5.0 % to about 20.0 % polyethylene glycol 6000 (PEG 6000), from about 50.0 to 65.0 % N-vinylcaprolactam and from about 15.0 % to about 45.0 % vinylacetate. It is preferred that the poly(N-vinyl caprolactam)-poly(vinyl acetate)-poly(ethylene glycol) graft copolymer is obtained or is obtainable from about 10.0 % to about 15.0 % polyethylene glycol 6000 (PEG 6000), from about 55.0 to 60.0 % *N-*vinylcaprolactam and from about 25.0 % to about 35.0 % vinylacetate, such as about 13.0 % PEG 6000, about 57.0 % *N*-vinylcaprolactam and about 30.0 % vinylacetate. The percentages refer to the % weight of the thermoresponsive polymer. Polyethylene glycol 6000 (PEG 6000) is commercially available and is known in the art.

The poly(*N*-vinyl caprolactam)-poly(vinyl acetate)-poly(ethylene glycol) graft copolymer may comprise, or consist essentially of, about 5.0 % to about 20.0 % of the first polymer unit, about 50.0 to 65.0 % of the second polymer unit and about 25.0 % to about 35.0 % of the third polymer unit. It is preferred that the poly(*N*-vinyl caprolactam)-poly(vinyl acetate)-poly(ethylene glycol) graft copolymer comprises, or consist essentially of, about 10.0 % to about 15.0 % of the first polymer unit, about 55.0 to 60.0 % of the second polymer unit and about 25.0 % to about 35.0 % of the third polymer unit, such as about 13.0 % of the first polymer unit, about 57.0 % of the second polymer unit and about 30.0 % of the third polymer unit. The percentages refer to the % weight of the thermoresponsive polymer.

The composition comprises 15.0 % w/w to 25.0 % w/w, even more preferably 17.5 % w/w to 22.5 % w/w of the polyvinyl caprolactam-polyvinyl acetate-polyethylene glycol graft copolymer (e.g. a poly(*N*-vinyl caprolactam)-poly(vinyl acetate)-poly(ethylene glycol) graft copolymer).

It is preferred that the composition comprises the polyvinyl caprolactam-polyvinyl acetate-polyethylene glycol graft copolymer (e.g. a poly(*N*-vinyl caprolactam)-poly(vinyl acetate)-poly(ethylene glycol) graft copolymer) as the only thermoresponsive polymer. Thus, no other thermoresponsive polymer may be present in the composition with the polyvinyl caprolactam-polyvinyl acetate-polyethylene glycol graft copolymer.

A commercially available poly(*N*-vinyl caprolactam)-poly(vinyl acetate)-poly(ethylene glycol) graft copolymer is Soluplus^{™} (BASF). Soluplus^{™} is commonly used as an additive in drug formulations to increase the bioavailability of poorly soluble drugs.

It has been found that the polyvinyl caprolactam-polyvinyl acetate-polyethylene glycol graft copolymer is a thermoresponsive polymer, which undergoes thermo-thickening (e.g. an increase in viscosity) behaviour with an increase in temperature. Surprisingly, this graft copolymer can undergo a sol-gel transformation at a physiological temperature, particularly when combined with a viscosity modifier in a relatively low amount.

The composition of the invention comprises a viscosity modifier.

For the avoidance of doubt, the viscosity modifier is different (e.g. a different compound) to the thermoresponsive polymer. Thus, the viscosity modifier does not, for example, comprise, or consist essentially of, a polyvinyl caprolactam-polyvinyl acetate-polyethylene glycol graft copolymer, such as the polyvinyl caprolactam-polyvinyl acetate-polyethylene glycol graft copolymer described herein.

Typically, a viscosity modifier may comprise, or consist essentially of, a poly(acrylic acid), a polyvinylpyrrolidone, a colloidal silica, an alginate, a microcrystalline cellulose, or a mixture of a microcrystalline cellulose and a carboxymethylcellulose. Poly(acrylic acid), polyvinylpyrrolidone, microcrystalline cellulose, and a mixture of a microcrystalline cellulose and a carboxymethylcellulose, are viscosity modifiers that are known in the art and are used as pharmaceutical excipients.

The composition comprises a total amount of 0.75 % w/w to 2.00 % w/w , such as a total amount of 1.00 % w/w to 2.00 % w/w, more preferably a total amount of 1.25 % w/w to 2.00 % w/w of viscosity modifier.

It is preferred that the viscosity modifier is a carbomer (e.g. Carbopol^{™}).

The poly(acrylic acid) may be a homopolymer of acrylic acid (e.g. Carbopol^{™} homopolymer) or a copolymer of acrylic acid (e.g. Carbopol^{™} copolymer or interpolymer). The copolymer of acrylic acid may be (i) a polymer of acrylic acid and a C₁₀-C₃₀ alkyl acrylate (e.g. Carbopol^{™} copolymer) or (ii) a polymer of acrylic acid comprising a block copolymer of polyethylene glycol and a C₁₀-C₃₀ alkyl acid ester (e.g. Carbopol^{™} interpolymer).

The poly(acrylic acid) is a cross-linked poly(acrylic acid). The poly(acrylic acid) may be cross-linked with a polyalkenyl polyether. The poly(acrylic acid) may be cross-linked with an allyl ether of propylene, an allyl ether of sucrose or an allyl ether of pentaerythritol.

The homopolymer of acrylic acid or the copolymer of acrylic acid may be cross-linked with a polyalkenyl polyether. The homopolymer of acrylic acid or the copolymer of acrylic acid may be cross-linked with an allyl ether of propylene, an allyl ether of sucrose or an allyl ether of pentaerythritol.

Poly(acrylic acid)s of the type described herein are known in the art and are commercially available (e.g. Carbopol^{™} polymer products, such as Ultrez 10 NF). Examples of poly(acrylic acid)s include Carbopol^{™} 71G, 971P, 974P, 980, 981, 5984, ETD 2020, Ultrez 10, 934, 934P, 940, 941 or 1342.

In general, a poly(acrylic acid), such as a Carbopol^{™} polymer product, having a CAS No. of 9003-01-4 is preferred (e.g. Carbopol^{™} 974P, 980NF or 934P NF).

It is preferred that the poly(acrylic acid) is cross-linked with a polyalkenyl polyether. More preferably, the poly(acrylic acid) is a homopolymer of acrylic acid cross-linked with a polyalkenyl polyether (e.g. Carbopol^{™} Ultrez 10 NF).Typically, the composition comprises a total amount of about 0.25 % w/w to about 5.00 % w/w of a poly(acrylic acid), such as a total amount of about 0.50 % w/w to about 3.50 % w/w, preferably a total amount of about 0.75 % w/w to about 2.75 % w/w (e.g. about 0.75 % w/w to about 2.00 % w/w), such as a total amount of about 1.00 % w/w to about 2.50 % w/w, more preferably a total amount of about 1.25 % w/w to about 2.25 % w/w, particularly of about 1.50 % w/w to about 2.00 % w/w.

Generally, the composition further comprises water. Thus, the composition may comprise, or consist essentially of, (a) 15.0 % w/w to 25.0 % w/w of a thermoresponsive polymer, (b) 0.75 % w/w to 2.00 % w/w of a viscosity modifier, and (c) water.

When the composition comprises components other than the thermoresponsive polymer and the viscosity modifier, or the composition consists essentially of the thermoresponsive polymer and the viscosity modifier, then water can make up the rest of the composition. Thus, in (c) above, the balance is water.

For example, the composition may consist essentially of (a) 15.0 % w/w to 25.0 % w/w of a thermoresponsive polymer, (b) 0.75 % w/w to 2.00 % w/w of a viscosity modifier, and (c) the balance is water.

In general, the composition of the invention does not comprise bismuth subnitrate (Bi₅O(OH)₉(NO₃)₄, preferably the composition does not comprise bismuth (i.e. bismuth in any form).

Typically, the composition of the invention does not comprise a heavy metal (i.e. a heavy metal in any form). The term "heavy metal" as used herein refers to a metal or a metalloid, where the elemental form of the metal or metalloid has a density greater than or equal to 3.50 g/cm³ at about 25°C (e.g. barium, which has a density of 3.62 g/cm³ at 25°C), preferably a density greater than or equal to 5.00 g/cm³) at about 25°C.

A heavy metal, such as bismuth, usually in the form of bismuth subnitrate, is included in many in internal teat seal formulations because of its high density. This high density helps to ensure that the formulation stays within the teat. The heavy metal particles can settle into the teat canal and/or the lower portion of the teat cistern and form a temporary physical barrier to seal off the teat canal and/or the lower portion of the teat cistern. A disadvantage of including a heavy metal in a teat seal formulation is there is a risk that the heavy metal will contaminate milk or dairy products. For example, bismuth from bismuth-containing teat seals is known to contaminate dairy products because it causes black spot defects (BSDs) in aged cheese.

In contrast to heavy-metal containing internal teat seal formulations, the composition of the invention can remain within the teat after application without the need for including a heavy-metal. Thus, the composition of the invention can avoid the contamination of dairy products associated with the presence of a heavy metal. This avoids the need to implement a withholding period of, for example, milk to ensure that it is free of the heavy metal.

The composition does not, in general, comprise barium (i.e. barium in any form).

Typically, the composition of the invention is non-toxic, particularly non-toxic to the animal, as defined herein.

It is generally preferred that the composition does not comprise chitosan.

The composition may further comprise a pH adjuster. Some of the viscosity modifiers described herein, such as the poly(acrylic acid), are acidic. It may be necessary for the composition to include a pH adjuster to ensure that the overall composition has a physiologically acceptable pH, such as neutral pH.

The pH adjuster may be an acid, a base or a buffer. It is preferred that the pH adjuster is a base or a buffer, more preferably the pH adjuster is a base.

When the pH adjuster is a base, then the pH adjuster may, for example, be selected from sodium hydroxide, potassium hydroxide, ammonium hydroxide, sodium bicarbonate, sodium carbonate, diethanolamine and monoethanolamine. It is preferred that the pH adjuster is sodium hydroxide.

In general, when the pH adjuster is an acid or a base, then the amount of pH adjuster added to the composition will depend on the desired pH level of the final product.

The composition may have a pH of about 5.0 to about 8.0, such as about 6.0 to about 7.5. When the pH adjuster is an acid or a base, then the amount of acid or base included in the composition is to provide a pH of about 5.0 to about 8.0, such as about 6.0 to about 7.5.

When the pH adjuster is a buffer, then the buffer may comprise a salt having a phosphate anion, a glycerophosphate anion or a mixture thereof. It is preferred that the buffer comprises one of (a) a phosphate anion or (b) a glycerophosphate anion.

The term "phosphate anion" as used herein includes an orthophosphate anion (e.g. PO₄³⁻), a polyphosphate anion, such as a diphosphate anion (e.g. P₂O₇⁴⁻) or a triphosphate anion (e.g. P₃O₁₀⁵⁻), or a conjugate acid thereof. It is preferred that the phosphate anion is an orthophosphate anion (e.g. PO₄³⁻) or a diphosphate anion (e.g. P₂O₇⁴⁻). More preferably, the phosphate anion is an orthophosphate anion (e.g. PO₄³⁻).

Typically, the buffer comprises a salt having a sodium cation, a potassium cation, a magnesium cation or a calcium cation. The sodium cation, potassium cation, magnesium cation or the calcium cation may be a counterion to the phosphate anion or the glycerophosphate anion. The overall charge of the salt is neutral (e.g. the stoichiometry of the cation balances the charge of the anion).

When the buffer comprises a glycerophosphate anion, it is preferred that the cation is a sodium cation or a potassium cation, preferably a sodium cation. It is preferred that the buffer is a sodium glycerophosphate.

When the buffer comprises a phosphate anion, it is preferred that the cation is a magnesium cation or a calcium cation, preferably a calcium cation. It is preferred that the buffer is a calcium phosphate.

In general, when the composition comprises a buffer (e.g. a pH adjuster), then the composition may comprise a total amount of less than or equal to about 2.5 % w/w (e.g. up to about 2.5 % w/w), such as about 0.1 % w/w to about 2.5 % w/w, preferably less than or equal to about 1.5 %w/w (e.g. up to about 1.5 % w/w), such as about 0.2 % w/w to about 1.5 % w/w.

The inclusion of a buffer, particularly a buffer comprising a salt having a phosphate anion or a glycerophosphate anion, can improve the storage stability of the composition by reducing or prevent phase separation. It is believed that the inclusion of the salt changes the chemical interaction between the thermoresponsive polymer and the viscosity modifier, thereby stabilising the composition.

The composition may comprise two or more pH adjusters. Thus, the composition may comprise a first pH adjuster and a second pH adjuster.

The first pH adjuster may be a base. For example, the first pH adjuster may be selected from sodium hydroxide, potassium hydroxide, ammonium hydroxide, sodium bicarbonate, sodium carbonate, diethanolamine and monoethanolamine. It is preferred that the first pH adjuster is sodium hydroxide.

The second pH adjuster may be a buffer, such as a buffer comprising a salt having a phosphate anion, a glycerophosphate anion or a mixture thereof, preferably a buffer as described above.

When the second pH adjuster is a buffer, then the composition may comprise the buffer in a total amount of less than or equal to about 2.5 % w/w (e.g. up to about 2.5 % w/w), such as about 0.1 % w/w to about 2.5 % w/w, preferably less than or equal to about 1.5 %w/w (e.g. up to about 1.5 % w/w), such as about 0.2 % w/w to about 1.5 % w/w.

Typically, the composition of the invention further comprises a tonicity modifier. The composition of the invention is preferably an isotonic composition. The purpose of the tonicity modifier is to adjust the tonicity of the composition to prevent osmotic shock at the site of administration, and thereby reduce local irritation.

The tonicity modifier may, for example, be selected from sodium chloride, potassium chloride, glycerin, mannitol, dextrose and trehalose. It is preferred that the tonicity modifier is sodium chloride or potassium chloride, more preferably sodium chloride.

The composition may further comprise a total amount of 0.1 % w/w to 2.5 % w/w of the tonicity modifier (e.g. preferably 0.1 % w/w to 1.0 % w/w, more preferably 0.20 % w/w to 0.75 % w/w), such as 0.25 % w/w to 2.25 % w/w, preferably 0.5 % w/w to 2.0 % w/w of the tonicity modifier.

In general, the composition may further comprise a preservative. Examples of preservatives include a paraben (e.g. methyl paraben, ethyl paraben, propyl paraben, butyl paraben), a phenol (e.g. phenol, meta cresol, chloro cresol), a quaternary ammonium compound (e.g. benzylalkonium chloride, benzethonium chloride), benzoic acid, sorbic acid or propylene glycol.

The composition may further comprise a cosolvent, preferably a single cosolvent.

Typically, the composition further comprises a total amount of about 0.5 % w/w to about 10.0 % w/w of the cosolvent, such as about 1.0 % w/w to about 7.5 % w/w, preferably 1.5 % w/w to about 5.0 % w/w of the cosolvent.

The cosolvent may be an alcohol, such as ethanol, ethylene glycol or propylene glycol. It is preferred that the cosolvent is propylene glycol. Advantageously, it has been found that the inclusion of an alcohol cosolvent can improve the storage stability of the composition by reducing or prevent phase separation. The thermoresponsive polymer, such as the polyvinyl caprolactam-polyvinyl acetate-polyethylene glycol graft copolymer, and the viscosity modifier may repel one another. It is believed that the inclusion of an alcohol cosolvent changes the chemical interaction between these ingredients, thereby stabilising the composition.

The composition of the invention may further comprise one or more excipients. The one or more excipients may be selected from a hydrophilic polymer, a pH responsive polymer, a cellulose or a cellulose derivative, a polysaccharide and a silica.

The hydrophilic polymer may be polyvinylpyrrolidone, a polyvinylpyrrolidone-vinyl acetate copolymer, a polyvinyl acetate (PVA), a polyvinyl acetate-acrylate, a polyethylene glycol (PEG), a poloxamer (e.g. Pluronic^{™} F127 and P123), a polysaccharide or an octablock star copolymer comprising terminal blocks having a poly(ethylene oxide) or a poly(propylene oxide) (e.g. Tetronic^{™}). When the viscosity modifier comprises, or consists essentially of, a hydrophilic polymer, then preferably the hydrophilic polymer is polyvinylpyrrolidone or a poloxamer, more preferably the hydrophilic polymer is polyvinylpyrrolidone. The pH response polymer may be an alginate, a hydroxypropyl methylcellulose acetate succinate (HPMC-AS), hyaluronic acid (HA) or a polymethacrylate. When the viscosity modifier comprises, or consists essentially of, a pH responsive polymer, then the pH responsive polymer is an alginate.

The cellulose or cellulose derivative may be ethyl cellulose; methyl cellulose (MC); a cellulose ether, such as hydroxypropyl cellulose (HPC), ethyl hydroxyethyl cellulose (EHEC) or hydroxypropyl methylcellulose (HPMC); a carboxymethylcellulose; or hydroxypropyl methylcellulose acetate succinate.

The polysaccharide may be a sulfated polysaccharide, such as a carrageenan; xanthan gum; tragacanth gum; guar gum; or agar.

The silica may be colloidal silica; fumed silica; or colloidal anhydrous silica.

Typically, the one or more excipients may be selected from polyvinylpyrrolidone (PVP), polyvinylpyrrolidone-vinyl acetate copolymer, an alginate, colloidal silica, carboxymethylcellulose and microcrystalline cellulose.

In general, the hydrophilic polymer may be included as a viscosity enhancer. Typically, the comprises a total amount of the viscosity enhancer that is less than the total amount of the viscosity modifier.

The composition may, in general, further comprise a colouring agent. The colouring agent may aid visibility of the composition during administration.

The composition may not comprise a therapeutic agent, such as a therapeutic agent for treating or preventing mastitis.

When the composition does not comprise a therapeutic agent for treating or preventing mastitis, then typically the composition is for use in the prevention of mastitis.

Alternatively, the composition may comprise a therapeutic agent, preferably a therapeutic agent is for treating or preventing mastitis.

Mastitis is often caused by a bacterial infection involving *Staphylococcal* species (e.g. *Streptococcus agalactiae, Staphylococcus aureus), Corynebacterium bovis, Mycoplasma,* coliforms (e.g. *Esherichia coli, Klebsiella spp., Enterobacter spp., Citrobacter spp*.), environmental *Streptococcal* species (e.g. *Strep. dysgalactiae, Strep. Uberis*), *Enterococcus spp., Pseudomonas spp.* etc. Inflammation can also be produced as a result of a viral infection, such as bovine herpesvirus II and IV or a paravaccinia virus (e.g. Pseudo Cowpox). The infection may be caused by atypical pathogens, such as a mycotic pathogen (e.g. *Candida spp., Aspergillus spp*.) or an algal microbe (e.g. *Prototheca spp*.)*.*

In general, the therapeutic agent is an antibacterial agent, an antiviral agent, an antimycotic agent, an antiparasitic agent or an immunostimulant agent.

The antibacterial agent is typically an antibiotic. The antibiotic may be selected from a macrolide antibiotic, a cephalosporin antibiotic, a lincosaminide antibiotic, a fluoroquinolone antibiotic, a tetracycline antibiotic, a penicillin antibiotic, an aminocyclitol antibiotic, a sulfonamide antibiotic, an aminoglycoside antibiotic and a chloramphenicol antibiotic.

The macrolide antibiotic may be tulathromycin, tildipirosin, tilmicosin, tylosin phosphate or gamithromycin. The cephalosporin antibiotic may be ceftiofur sodium, ceftiofur hydrochloride, ceftiofur crystalline free acid, cefovecin sodium or cefpodoxime proxetil. The lincosaminide antibiotic may be lincomycin, piriimycin hydrochloride or clindamycin hydrochloride. The fluoroquinolone antibiotic may be danofloxacin, enrofloxacin or marbofloxacin. The tetracycline antibiotic may be tetracycline, chlortetracycline, oxytetracycline or doxycycline. The penicillin antibiotic may be pencillin, ampicillin, cloxacillin or amoxicillin trihydrate, where the penicillin antibiotic may optionally be used in combination with clavulanic acid. The aminocyclitol antibiotic may be spectinomycin. The sulfonamide antibiotic may be trimethoprim/sulfadiazine or sulfadimethoxine/ormetoprim. The aminoglycoside antibiotic may be streptomycin. The chloramphenicol antibiotic may be chloramphenicol, thiamphenicol or florfenicol.

When the therapeutic agent is for treating or preventing mastitis, then the antibiotic is preferably selected from streptomycin, ampicillin, cloxacillin, penicillin and tetracycline.

The therapeutic agent may be a bacteriocin, such as nisin or lacticin.

Additionally or alternatively, the therapeutic agent may be an antiseptic. The antiseptic may be selected from povidone-iodine, chlorhexidine or a quaternary ammonium compound, such as cetrimide or benzalkonium chloride (BZK).

The invention provides a system for administering the composition to mammary gland of an animal. The animal may be a non-human animal. The system is preferably for administering the composition to a mammary gland of a female, non-human mammal.

The system comprises (i) an application device and/or an infusion device, and (ii) the composition. The application device or the infusion device may be filled or pre-loaded with the composition. Application devices or infusion devices are known in the art.

The application device may be a spray gun or a shower head. The infusion device may be a cannula or a syringe, particularly a syringe with a teat needle or an intramammary tip. It is preferred that the system comprise an infusion device, which is preferably a syringe.

The system, the package or the kit of parts of the invention may comprise a unit dose of the composition.

The invention also relates to the composition for use in a method method of treating or preventing mastitis.

Any reference to the "treatment or prevention of mastitis" or to "treating or preventing mastitis" used herein relates to mastitis in an animal, preferably a female animal. The animal may be a non-human animal, and is preferably a female, non-human mammal.

It is preferred that the animal belongs to the family Bovidae. The animal belonging to the family Bovidae is preferably selected from a cow, a goat, a sheep and a water buffalo. More preferably, the animal is a cow, a goat or a water buffalo. The animal is most preferably a cow.

The animal may be a dairy animal, such as a heifer or a cow.

In general, the composition of the invention is for topical administration and/or for administration by infusion, preferably for topical administration to a mammary gland of the animal and/or for administration by infusion into the mammary gland of the animal.

Typically, the composition is administered at the start of the dry period, during the drying off period or prior to first calving in primiparous heifers. It is preferred that the composition is administered at the start of the dry period. The dry period is the period where milking for the lactation season has ceased as the animals, particularly cows, have no milk to expel and are therefore "dry".

Generally, a therapeutically effective amount of the composition is administered to the animal. The term "therapeutically effective amount" refers to an amount of the composition that, when administered to an animal for treating or preventing mastitis, is sufficient to affect such treatment or prevention of the disease.

The therapeutic agent may be administered as part of the composition or in a different composition intended for separate, simultaneous, concomitant or sequential administration by the same or a different route.

The amount of therapeutic agent required to achieve a therapeutic effect will vary with the active ingredient, the route of administration and the animal being treated.

The composition is for use in a method method of treating or preventing mastitis, wherein said method comprises administering the composition to a mammary gland of an animal. The method may further comprise administering a therapeutic agent, such as defined herein, separately to, simultaneously with, concomitantly with or sequentially to the composition.

A method of manufacturing the composition of the invention is also described. The method comprises mixing a suspension of a thermoresponsive polymer with a viscosity modifier. It is preferred that the method comprises mixing a suspension of the thermoresponsive polymer with a suspension of the viscosity modifier.

The amounts of thermoresponsive polymer and viscosity modifier will depend on the final amount of each ingredient as defined by the claims.

The other ingredients of the composition, such as a pH adjuster, a tonicity modifier etc, may be added to the suspension comprising the thermoresponsive polymer and the viscosity modifier.

The suspension of the thermoresponsive polymer may be mixed with a viscosity modifier and a colouring agent.

### EXAMPLES

The invention is further illustrated by the following examples which should not be construed as a limiting.

### Example 1

### Preparative method

The following materials were used to manufacture the compositions below.

**Table 1**

| Material | CAS No. |
|---|---|
| Poly(N-vinylcaprolactam)-poly(vinyl acetate)-polyethylene glycol | 402932-23-4 |
| Poly(acrylic acid) | 195739-91-4 |
| Sodium hydroxide | - |
| Sodium chloride | - |
| Water | - |

Compositions were prepared using a method to ensure homogeneity.

75 g of a poly(N-vinylcaprolactam)-poly(vinyl acetate)-polyethylene glycol graft copolymer (referred to below as the "graft copolymer") was slowly added to 150 mL of water whilst continually mixing.

A separate suspension was prepared by slowly adding 5 g a poly(acrylic acid) viscosity modifier to 100 mL of water whilst mixing. The poly(acrylic acid) was a polymer of acrylic acid cross-linked with a polyalkenyl polyether.

The separate suspensions of the graft copolymer and the poly(acrylic acid) were combined together by mixing in a beaker in sufficient quantities to provide the required amount of each component in the final composition. See Table 2 below.

100 mL of 10 %w/v NaOH was made up using NaOH pellets. Whilst mixing, a pH reading was taken of the combined polymer mixture. The 10 %w/v NaOH was added to the mixture until a target pH of 6.5 was obtained.

The compositions in Table 2 were prepared using this method.

**Table 2**

| Formulation No. | graft copolymer (% w/w) | poly(acrylic acid) (% w/w) | water (% w/w) |
|---|---|---|---|
| 1 | 14.02 | 1.87 | 84.11 |
| 2 | 18.69 | 1.87 | 79.44 |
| 3 | 16.47 | 1.56 | 81.97 |
| 4 | 18.81 | 1.56 | 79.63 |
| 5 | 17.06 | 0.98 | 81.96 |
| 6 | 21.14 | 0.98 | 77.88 |
| 7 | 15.19 | 0.70 | 84.11 |
| 8 | 23.36 | 0.70 | 75.94 |
| 9 | 23.36 | 1.12 | 75.52 |

### Results

A study was performed to measure the temperature-dependent behaviour at constant applied stress conditions. The values of elastic and storage moduli as a function of time at a constant stress of 0.1 - 1.0 Pa (or another appropriate value in the plateau region of LVE) and frequency (f = 0.1 - 10 Hz) were assessed. In the first step of the experiment (5 mins), the temperature was kept at 25°C. In the second step, the temperature was increased at approximately 10°C/min to 37°C. In the final step (5 - 10 min), the temperature was fixed at 37°C.

The results are expressed as elastic modulus G' and loss tangent (tan δ). The results are shown in Table 3 at the temperatures 25°C and 37°C. For compositions that are more solid than liquid tan δ < 1, whereas liquids have a tan δ > 1. Another measure of the effect of temperature increase on the solid nature of the composition is the ratio of G'(37°C)/G'(25°C). See the column headed "G' ratio" in Table 3. This is complemented by the tan δ 25°C/tan δ 37°C ratio, which provides insight into the flowing nature of the product. See the column headed "Tan δ ratio" in Table 3.

**Table 3**

| Formulation No. | G' 25°C (Pa) | Tan δ 25°C | G' 37°C (Pa) | Tan δ 37°C | G' ratio (37°C/25°C) | Tan δ ratio (25°C/37°C) |
|---|---|---|---|---|---|---|
| 1 | 399 | 0.51 | 1450 | 0.32 | 3.63 | 1.57 |
| 2 | 286 | 0.71 | 1433 | 0.40 | 5.01 | 1.80 |
| 3 | 228 | 0.76 | 1387 | 0.37 | 6.07 | 2.03 |
| 4 | 179 | 0.98 | 1362 | 0.43 | 7.60 | 2.28 |
| 5 | 53.8 | 0.99 | 951 | 0.43 | 17.7 | 2.34 |
| 6 | 43.7 | 1.65 | 929 | 0.51 | 21.2 | 3.22 |
| 7 | 2.25 | 2.35 | 212 | 0.70 | 93.8 | 3.37 |
| 8 | 8.75 | 3.36 | 479 | 0.68 | 54.7 | 4.98 |
| 9 | 30.6 | 2.42 | 742 | 0.62 | 24.3 | 3.90 |

The rheological characteristics of all of the compositions provided significant increases in resistance to deformation (storage modulus G') as well as decreased flowing nature (tan δ) at higher temperatures. This indicates all formulations exhibit favourable changes in rheological behaviour on temperature increase.

Within the compositions measured, the characteristic thermoresponsive nature of the graft copolymer was maintained.

At 37°C, the thermoresponsive nature of the graft copolymer has a significant impact when comparing against those values seen at 25°C. The highest values of storage modulus were found at high poly(acrylic acid) concentrations but lower graft copolymer values. This finding demonstrates that the larger proportion of the graft copolymer present acts to reduce the overall storage modulus, even at higher temperatures regardless of the thermoresponsive nature.

The relative increase in storage modulus (G') and reduction in tan δ were determined by the measures of G'(37°C)/G'(25°C) and tan δ 25°C/tan δ 37°C, with higher values in both measures indicating a greater favourable change in the composition properties on temperature increase. Higher values of the ratio G'(37°C)/G'(25°C) indicate a more significant reduction in flow, with values of less than 1 indicating an increase of flow on temperature increase. The greatest changes were exhibited in gels where the amount of the graft copolymer was high and the amount of the poly(acrylic acid) was low. The greater relative increase in storage modulus at higher graft copolymer concentrations indicates that the thermoresponsive effect is enhanced by greater graft copolymer inclusion.

### Example 2

### Comparison with bismuth-containing composition

A composition was prepared in accordance with the invention using the method described in Example 1. The composition contained 30 % w/w of a poly(N-vinylcaprolactam)-poly(vinyl acetate)-polyethylene glycol graft copolymer, 2.00 % w/w of a poly(acrylic acid) viscosity modifier and 0.15 % w/w of NaCl.

A study was performed to determine the rheological properties of the composition and the rheological properties of a commercially available bismuth-containing teat seal formulation. This commercially available teat seal formulation contains bismuth subnitrate (65 % wt.), aluminium di-/tri stearate, povidone-iodine and liquid paraffin.

The values of elastic and storage moduli as a function of time at a constant stress of 1.0 Pa and frequency (f = 1.0 Hz) were assessed. In the first step of the experiment (5 mins), the temperature was constant at 25°C. In the second step the temperature was increased at approximately 10°C/min to 37°C. In the final step (5 - 10 min), the temperature was constant at 37°C.

### Results

The results are shown in Figure 1. The results show that the composition of the invention has improved rheological characteristics at higher temperature relative to its performance at lower temperatures in comparison to the commercially available bismuth-containing formulation. The data demonstrate that the commercially available formulation shows reduced performance at higher temperatures. The composition of the invention shows slightly less solidity than the commercially available formulation at both temperatures, which is advantageous for administering the composition by syringe. The solidity of the formulations is not the only factor influencing retention within the teat. Adhesive forces between the formulation and the mucosa may also play a significant role.

### Example 3 (comparative compositions)

The compositions in Tables 4 to 6 were also prepared.

**Table 4**

| Material | Brand/Grade |
|---|---|
| Poly(N-vinylcaprolactam)-poly(vinyl acetate)-polyethylene glycol | CAS No. 402932-23-4 |
| Carboxymethyl cellulose/Microcrystalline cellulose | Avicel^{™} RC591 |
| Sodium chloride | N/A |
| Water | N/A |

**Table 5**

| Material | Brand/Grade |
|---|---|
| Poly(N-vinylcaprolactam)-poly(vinyl acetate)-polyethylene glycol | CAS No. 402932-23-4 |
| Colloidal silica | Cab-o-sil^{™} |
| Sodium chloride | N/A |
| Water | N/A |

**Table 6**

| Material | Brand/Grade |
|---|---|
| Poly(N-vinylcaprolactam)-poly(vinyl acetate)-polyethylene glycol | CAS No. 402932-23-4 |
| Polyvinyl pyrrolidone | PVP (Kollidon^{™} K30) |
| Sodium chloride | N/A |
| Water | N/A |

### Example 4

The compositions in Tables 7 to 9 were also prepared.

**Table 7**

| Material | Brand/Grade | Quantity (% w/w unless stated) |
|---|---|---|
| Poly(N-vinylcaprolactam)-poly(vinyl acetate)-polyethylene glycol | CAS No. 402932-23-4 | 19.5 |
| Poly(acrylic acid) | CAS No. 9003-01-4 | 1.85 |
| NaOH 10% w/v | N/A | QS to pH 6 |
| Calcium phosphate/dicalcium phosphate | N/A | ~0.001M |
| Sodium chloride | N/A | 0.73 |
| Water | N/A | balance to 100% |

**Table 8**

| Material | Brand/Grade | Quantity (% w/w unless stated) |
|---|---|---|
| Poly(N-vinylcaprolactam)-poly(vinyl acetate)-polyethylene glycol | CAS No. 402932-23-4 | 19.5 |
| Poly(acrylic acid) | CAS No. 9003-01-4 | 1.85 |
| NaOH 10% w/v | N/A | QS to pH 6 |
| Sodium glycerophosphate | N/A | 0.04M |
| Sodium chloride | N/A | 0.27 |
| Water | N/A | balance to 100% |

**Table 9**

| Material | Brand/Grade | Quantity (% w/w unless stated) |
|---|---|---|
| Poly(N-vinylcaprolactam)-poly(vinyl acetate)-polyethylene glycol | CAS No. 402932-23-4 | 19.5 |
| Poly(acrylic acid) | CAS No. 9003-01-4 | 1.85 |
| NaOH 10% w/v | N/A | QS to pH 6 |
| Propylene glycol | N/A | 3.33 |
| Sodium chloride | N/A | 0.27 |
| Water | N/A | balance to 100% |

The formulations in Tables 7 to 9 were found to have improved storage ability because they did not undergo phase separation after several months. This improvement in storage stability is due to the presence of the calcium phosphate/dicalcium phosphate, the sodium glycerophosphate or the propylene glycol.

## Claims

1. A composition comprising:
(a) 15.0 % w/w to 25.0 % w/w of a polyvinyl caprolactam-polyvinyl acetate-polyethylene glycol graft copolymer; and
(b) 0.75 % w/w to 2.00 % w/w of a viscosity modifier, wherein the viscosity modifier is a cross-linked poly(acrylic acid).

2. The composition according to claim 1, wherein the cross-linked poly(acrylic acid) is a polymer of acrylic acid cross-linked with a polyalkenyl polyether.

3. The composition according to claim 1 or claim 2, wherein the poly(acrylic acid) is a cross-linked with an allyl ether of propylene, an allyl ether of sucrose or an allyl ether of pentaerythritol.

4. The composition according to any one of claims 1 to 3, wherein the poly(acrylic acid) is a homopolymer of acrylic acid or a copolymer of acrylic acid, preferably the copolymer of acrylic acid is (i) a polymer of acrylic acid and a C₁₀-C₃₀ alkyl acrylate or (ii) a polymer of acrylic acid comprising a block copolymer of polyethylene glycol and a C₁₀-C₃₀ alkyl acid ester.

5. The composition according to any one of the preceding claims, wherein the polyvinyl caprolactam-polyvinyl acetate-polyethylene glycol graft copolymer has a weight average molecular weight of from 50000 to 250000 g.mol⁻¹.

6. The composition according to any one of the preceding claims, which does not comprise bismuth.

7. The composition according to any one of the preceding claims, further comprising a pH adjuster, preferably wherein:
(a) the pH adjuster is a buffer, preferably a buffer comprising a salt having a phosphate anion, a glycerophosphate anion or a mixture thereof; and/or
(b) the pH adjuster is a base, preferably selected from sodium hydroxide, potassium hydroxide, ammonium hydroxide, sodium bicarbonate, sodium carbonate, diethanolamine and monoethanolamine.

8. The composition according to any one of the preceding claims, further comprising a total amount of 0.1 % w/w to 2.5 % w/w of a tonicity modifier.

9. The composition according to any one of the preceding claims, further comprising a cosolvent, preferably wherein the cosolvent is propylene glycol.

10. The composition according to any one of the preceding claims, further comprising a therapeutic agent.

11. The composition according to any one of the preceding claims, further comprising water, preferably wherein the balance is water.

12. A composition according to any one of the preceding claims for use in therapy and/or for use as a medicament.

13. A composition according to any one of the preceding claims for use in the treatment or prevention of mastitis.

14. A method of manufacturing a composition as defined in any one of claims 1 to 11, wherein the method comprises mixing a suspension of a polyvinyl caprolactam-polyvinyl acetate-polyethylene glycol graft copolymer with a viscosity modifier and optionally a colouring agent, wherein the viscosity modifier is a cross-linked poly(acrylic acid).

## Patentansprüche

1. Zusammensetzung, umfassend:
(a) 15,0 Gew.-% bis 25,0 Gew.-% eines Polyvinylcaprolactam-Polyvinylacetat-Polyethylenglykol-Pfropfcopolymers; und
(b) 0,75 Gew.-% bis 2,00 Gew.-% eines Viskositätsmodifikators, wobei der Viskositätsmodifikator eine vernetzte Poly(acrylsäure) ist.

2. Zusammensetzung nach Anspruch 1, wobei die vernetzte Poly(acrylsäure) ein Polymer einer Acrylsäure ist, die mit einem Polyalkenylpolyether vernetzt ist.

3. Zusammensetzung nach Anspruch 1 oder Anspruch 2, wobei die Poly(acrylsäure) mit einem Allylether von Propylen, einem Allylether von Saccharose oder einem Allylether von Pentaerythritol vernetzt ist.

4. Zusammensetzung nach einem der Ansprüche 1 bis 3, wobei die Poly(acrylsäure) ein Homopolymer von Acrylsäure oder ein Copolymer von Acrylsäure ist, wobei vorzugsweise das Copolymer von Acrylsäure (i) ein Polymer von Acrylsäure und einem C₁₀-C₃₀-Alkylacrylat oder (ii) ein Polymer von Acrylsäure ist, das ein Blockcopolymer von Polyethylenglykol und einem C₁₀-C₃₀-Alkylsäureester umfasst.

5. Zusammensetzung nach einem der vorhergehenden Ansprüche, wobei das Polyvinylcaprolactam-Polyvinylacetat-Polyethylenglykol-Pfropfcopolymer ein gewichtsmittleres Molekulargewicht von 50000 bis 250000 g.mol⁻¹ aufweist.

6. Zusammensetzung nach einem der vorhergehenden Ansprüche, die kein Wismut umfasst.

7. Zusammensetzung nach einem der vorhergehenden Ansprüche, ferner umfassend ein pH-Einstellmittel, wobei vorzugsweise:
(a) das pH-Einstellmittel ein Puffer ist, vorzugsweise ein Puffer, der ein Salz mit einem Phosphatanion, einem Glycerophosphatanion oder einer Mischung davon umfasst; und/oder
(b) das pH-Einstellmittel eine Base ist, vorzugsweise ausgewählt aus Natriumhydroxid, Kaliumhydroxid, Ammoniumhydroxid, Natriumbicarbonat, Natriumcarbonat, Diethanolamin und Monoethanolamin.

8. Zusammensetzung nach einem der vorhergehenden Ansprüche, ferner umfassend eine Gesamtmenge von 0,1 Gew.-% bis 2,5 Gew.-% eines Tonizitätsmodifikators.

9. Zusammensetzung nach einem der vorhergehenden Ansprüche, ferner umfassend ein Hilfslösungsmittel, wobei das Hilfslösungsmittel vorzugsweise Propylenglykol ist.

10. Zusammensetzung nach einem der vorhergehenden Ansprüche, ferner umfassend ein therapeutisches Mittel.

11. Zusammensetzung nach einem der vorhergehenden Ansprüche, ferner umfassend Wasser, wobei vorzugsweise der Rest Wasser ist.

12. Zusammensetzung nach einem der vorhergehenden Ansprüche zur Verwendung in der Therapie und/oder zur Verwendung als ein Medikament.

13. Zusammensetzung nach einem der vorhergehenden Ansprüche zur Verwendung bei der Behandlung oder Vorbeugung von Mastitis.

14. Verfahren zur Herstellung einer Zusammensetzung wie in einem der Ansprüche 1 bis 11 definiert, wobei das Verfahren Mischen einer Suspension eines Polyvinylcaprolactam-Polyvinylacetat-Polyethylenglykol-Pfropfcopolymers mit einem Viskositätsmodifikator und optional einem Färbemittel umfasst, wobei der Viskositätsmodifikator eine vernetzte Poly(acrylsäure) ist.

## Revendications

1. Composition comprenant :
(a) 15,0 % p/p à 25,0 % p/p d'un copolymère greffé polyvinyle caprolactame-acétate de polyvinyle-polyéthylène glycol ; et
(b) 0,75 % p/p à 2,00 % p/p d'un modificateur de viscosité, dans laquelle le modificateur de viscosité est un poly(acide acrylique) réticulé.

2. Composition selon la revendication 1, dans laquelle le poly(acide acrylique) réticulé est un polymère d'acide acrylique réticulé avec un polyéther polyalcényle.

3. Composition selon la revendication 1 ou la revendication 2, dans laquelle le poly(acide acrylique) est réticulé avec un éther allylique de propylène, un éther allylique de saccharose ou un éther allylique de pentaérythritol.

4. Composition selon l'une quelconque des revendications 1 à 3, dans laquelle le poly(acide acrylique) est un homopolymère d'acide acrylique ou un copolymère d'acide acrylique, de préférence le copolymère d'acide acrylique est (i) un polymère d'acide acrylique et un acrylate d'alkyle en C₁₀-C₃₀ ou (ii) un polymère d'acide acrylique comprenant un copolymère séquencé de polyéthylène glycol et d'un ester d'acide alkyle en C₁₀-C₃₀.

5. Composition selon l'une quelconque des revendications précédentes, dans laquelle le copolymère greffé caprolactame de polyvinyle-acétate de polyvinyle-polyéthylène glycol a un poids moléculaire moyen en poids de 50 000 à 25 0000 g.mol⁻¹.

6. Composition selon l'une quelconque des revendications précédentes, qui ne comprend pas de bismuth.

7. Composition selon l'une quelconque des revendications précédentes, comprenant en outre un ajusteur de pH, de préférence dans laquelle :
(a) l'ajusteur de pH est un tampon, de préférence un tampon comprenant un sel ayant un anion phosphate, un anion glycérophosphate ou un mélange de ceux-ci ; et/ou
(b) l'ajusteur de pH est une base, de préférence choisie parmi l'hydroxyde de sodium, l'hydroxyde de potassium, l'hydroxyde d'ammonium, le bicarbonate de sodium, le carbonate de sodium, la diéthanolamine et la monoéthanolamine.

8. Composition selon l'une quelconque des revendications précédentes, comprenant en outre une quantité totale de 0,1 % p/p à 2,5 % p/p d'un modificateur de tonicité.

9. Composition selon l'une quelconque des revendications précédentes, comprenant en outre un cosolvant, de préférence dans laquelle le cosolvant est le propylène glycol.

10. Composition selon l'une quelconque des revendications précédentes, comprenant en outre un agent thérapeutique.

11. Composition selon l'une quelconque des revendications précédentes, comprenant en outre de l'eau, de préférence dans laquelle le reste est de l'eau.

12. Composition selon l'une quelconque des revendications précédentes destinée à être utilisée en thérapie et/ou à être utilisée en tant que médicament.

13. Composition selon l'une quelconque des revendications précédentes destinée à être utilisée dans le traitement ou la prévention d'une mammite.

14. Procédé de fabrication d'une composition telle que définie dans l'une quelconque des revendications 1 à 11, dans lequel le procédé comprend le mélange d'une suspension d'un copolymère greffé polyvinyle caprolactame-polyvinyle acétate-polyéthylène glycol avec un modificateur de viscosité et éventuellement un agent colorant, dans lequel le modificateur de viscosité est un poly(acide acrylique) réticulé.
